(19) Europäisches Patentamt
European Patent Office
Office européen des brevets

(11) **EP 3 108 835 A1**

(12) **EUROPEAN PATENT APPLICATION**

(43) Date of publication:
**28.12.2016 Bulletin 2016/52**

(51) Int Cl.:
***A61B 17/88*** (2006.01)   ***A61F 2/46*** (2006.01)

(21) Application number: **15305974.6**

(22) Date of filing: **24.06.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(71) Applicants:
• **Université de Strasbourg**
  **67000 Strasbourg (FR)**
• **CENTRE NATIONAL DE**
  **LA RECHERCHE SCIENTIFIQUE -CNRS-**
  **75794 Paris Cedex 16 (FR)**
• **Institut National Des Sciences**
  **Appliquees**
  **67000 Strasbourg (FR)**
• **Université Paris Sud**
  **91400 Orsay (FR)**

(72) Inventors:
• **Schmitt, François**
  **67000 Strasbourg (FR)**
• **Meylheuc, Laurence**
  **67130 Bergbieten (FR)**
• **Bayle, Bernard**
  **67000 Strasbourg (FR)**
• **Lepoutre, Nicole**
  **67100 Strasbourg (FR)**
• **Lefeuvre, Élie**
  **93100 Montreuil (FR)**
• **Martincic, Émile**
  **94800 Villejuif (FR)**
• **Valls, Aida**
  **91400 Orsay (FR)**

(74) Representative: **Novagraaf Technologies**
  **Bâtiment O2**
  **2, rue Sarah Bernhardt**
  **CS90017**
  **92665 Asnières-sur-Seine Cedex (FR)**

(54)    **INJECTION DEVICE OF BONE CEMENT FOR PERCUTANEOUS VERTEBROPLASTY**

(57)    The object of the present invention is an injection device (1) of curing cement (12) for percutaneous vertebroplasty, said device(1) comprising a system (7,8) for generating volumetric flow of said cement (12), and a pipe (17) connecting said injection device (1) to a percutaneous needle (14), said injection device (1) being characterized in that it further comprises at least one active heat exchanger(s) (13) located on the pipe (17) for dynamic controlled heating and/or cooling of said cement (12) during the injection.

FIG. 1

## Description

## TECHNICAL FIELD OF THE INVENTION

The present invention relates to an injection device of curing cement in the field of percutaneous vertebroplasty interventions.

Percutaneous vertebroplasty is a non-surgical minimal invasive intervention that involves injecting bone cement into the vertebral body of a human being under medical imaging control, to reinforce or restructure a weakened or broken vertebra. Such an intervention thus allows the stabilization of the vertebrae and the reduction of severe pain for people suffering from vertebral compression fractures, most often caused by osteoporosis but also, less frequently, by metastases or traumatic fractures[1].

In order to achieve this, a known method consists in injecting a medical grade self-solidifying compound (cement), generally a mixture of methyl methacrylate (MMA) and polymethacrylate (PMMA) and a contrast agent. The cement is injected through a large section needle whose distal part is placed inside the vertebral spongious body. Such an intervention is usually performed under fluoroscopic guidance in order to prevent cement leakage that could lead to several handicapping or lethal complications to the patient. The imaging device emits harmful ionizing radiations that may affect the physician who realizes the intervention.

In that context, the Applicant has developed a tool that provides the practitioner with an enhanced injection control while being as far as necessary from the X-ray source to reduce his/her exposure. This tool consists in:

- an injection device that generates the force necessary to inject the cement, comprising a thermal regulation system that allows the dynamic control of the viscosity of the cement through its thermo-dependency,
- a master device that remotely controls the injection device.

It is known by the skilled person that thermal solutions can be implemented, that use passive cooling of their device in order to slow the reaction for pre-mixed syringes waiting to be used, or active heating in order to increase the speed of solidification and give the cement a higher viscosity to decrease leakage risk.

However, in these known devices, the cooling of the cement is limited to a pre- or per-operative conservation of its fluidity before the actual injection[2],[3]. The cooling of the cement can also be limited to protect the patient from a potential burn caused by the needle[4]. Furthermore, it is also known by the man skilled in the art to use heating of the cement to increase its viscosity[4] but the heating is not dynamically controlled. At last, even if the injection device of US 8,523,871[4] implements both heating and cooling functions, only the actual heating can be controlled because of the positioning of the sensor.

## SUMMARY OF THE INVENTION

The invention therefore proposes an instrumented injection device that prevents all the aforementioned drawbacks, notably by allowing a dynamic controlled heating and/or cooling of said cement during the injection that leads to a dynamic control of the viscosity of the cement during the injection.

More particularly, the present invention relates to an injection device of curing cement for percutaneous vertebroplasty, said device comprising a system for generating volumetric flow of said cement, and a pipe connecting said injection device to a percutaneous needle, said injection device being characterized in that it further comprises at least one active heat exchanger(s) located on the pipe for dynamic controlled heating and/or cooling of said cement during the injection.

By active heat exchanger, is meant according to the present invention, a heat exchanger whose operation in heating or cooling is controlled by a supply of external energy.

Contrary to the teaching of US patent US 8,523,871[4] (where even if both heating and cooling function are implemented, only the actual heating can be controlled), the active heat exchanger of the injection device of the invention allows a precise, dynamic and full control of the temperature of the cement in a given section of the pipe in order to follow a viscosity setpoint $\eta^*$, evolving over time in a given interval [$\eta_{min}$, $\eta_{max}$]. This control is achieved by:

- accelerating the curing reaction by heating if the viscosity of the cement is lower than $\eta^*$;
- slowing down the curing reaction by cooling if the viscosity of the cement is higher than $\eta^*$.

As the cement itself acts as a thermal insulator, it is possible to regulate its temperature on reduced pipe diameters and therefore it is less convenient to do it on the syringe as claimed in the US patent application US2013/0190680[2].

The system for generating volumetric flow of the device of the present invention may advantageously comprise a syringe for containing the cement, and a piston, that is movable inside the syringe for pushing the cement inside the pipe through the syringe outlet.

According to an embodiment of the invention, the said active heat exchanger(s) located along the pipe may comprise a thermal block with at least one Peltier module mounted on the pipe. Preferably, the thermal block may comprise:

a central regulated block made out of a thermal conducting material with low thermal inertia,

- at least one stack on the regulated block and including:

    • a Peltier module,
    • at least one heat sink made out of thermal conducting material with low thermal inertia,
    • a fan,

- a thermal insulation wrapping the central block, and
- at least one temperature sensor.
- a thermal insulation wrapping the central block, to improve the efficiency of the exchanger, and
- at least one temperature sensor.

According to another embodiment of the invention, the injection device of the invention may also further comprise a deported active heat exchanger put on a closed fluid circuit with a fluid-to-cement heat exchanger. The deported active heat exchanger of the invention operates in cooling and/or in heating, in order to reduce the exchanger's footprint at the proximity of the patient.

Preferably, the deported active heat exchanger of the invention may also comprise at least one Peltier module.

Advantageously, in this deported embodiment, the heat transfer fluid flowing between the deported active heat exchanger and the fluid-to-cement exchanger may be a liquid, a gas or a mixture of liquid and gas, and preferably water.

Besides the active heat exchanger (s) located on the pipe, the injection device of the invention may also advantageously further comprise at least one heat exchanger on the syringe notably to cool the cement contained inside the syringe before being injected in the vertebra.

Advantageously, the heat exchanger on the syringe may be a passive heat exchanger, in order to keep the physical properties of the cement as constant as possible through the injection when not circulating in the pipe. Preferably, the passive heat exchanger located on the syringe may comprise a sheath surrounding the syringe that is preferably filled with a eutectic fluid such as a gel.

Advantageously, the said active and/or passive heat exchangers may be removable. Advantageously, the injection device may also comprise a pressure sensor presenting a sensing area, said pressure sensor being located on a defined point of the cement pipe. Preferably, the sensing area of said pressure sensor may not be in direct contact with the cement.

Advantageously, the pressure of the cement in the pipe may be transmitted to the sensing area of said pressure sensor by an intermediary incompressible material, preferably water or an elastomeric material. Preferably, the cement pipe further may further comprise a sterile elastomeric membrane, able to transmit the cement pressure to said pressure sensor.

The injection device of the invention presents the major advantages:

- to give the physician an enhanced control over the injection procedure,
- to give the physician a pretty complete information concerning the cement and the injection state, that allows the injection of the cement at a high viscosity in order to reduce the leakage risk, and
- to optimize the cement working period.
- to remotely monitor intra vertebral pressure

## BRIEF DESCRIPTION OF THE DRAWINGS

Other features and advantages of the invention will become more clearly apparent on reading the following description, given with reference to the appended figures, which illustrate non-limiting examples of preferred embodiments:

- FIG. 1 represents a tridimensional CAD general view of the entire injection device of the invention as a whole,
- FIG. 2 represents a schematic diagram of the injection device illustrated on figure 1,
- FIG. 3 represents a CAD view of a master device that remotely controls the injection,
- FIG. 4A represents a tridimensional CAD view of a heat exchanger with a thermal block,
- FIG. 4B represents its corresponding cross-sectional schematic view,
- FIG. 5 represents a principle diagram of a deported active heat exchanger put on a closed fluid circuit with a fluid-to-cement heat exchanger, according to an embodiment of the invention,
- FIG. 6 represents a detailed view of the deported active heat exchanger illustrated on FIG. 5,
- FIG. 7 represents a detailed view of the water-to-cement heat exchanger illustrated on FIG. 5,
- FIG. 8 represents an exploded view of a sheath surrounding the syringe, according to an embodiment of the invention,
- FIG. 9 represents a detailed view of the syringe with sheath illustrated on FIG. 8,
- FIG. 10 represents a cross-sectional schematic view of an embodiment of a pressure sensor (located on the cement pipe) with a water-filled channel,
- FIG. 11 represents a cross-sectional schematic view of another embodiment of a pressure sensor with an elastomer-filled channel,
- FIG. 12 represents a cross-sectional schematic view of another embodiment of a pressure sensor with both an elastomer-filled channel and an elastomeric membrane.

For the sake of clarity, identical or similar elements have been referenced with identical reference symbols in all the figures.

## DETAILED DESCRIPTION OF THE PREFERRED EMBODIMENTS

For purposes of understanding the principles of the invention, reference will now be made to the embodiments illustrated in the drawings and the accompanying text.

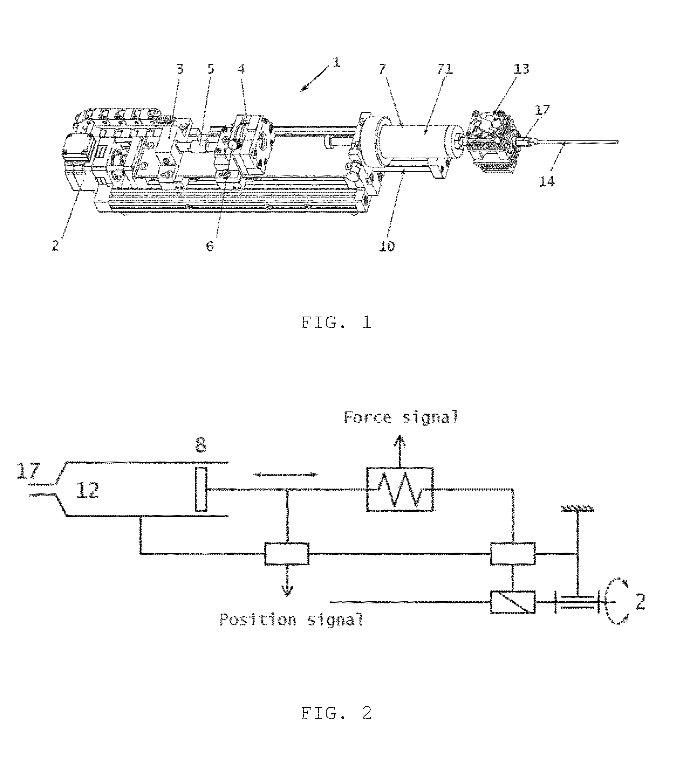

As shown in FIG. 1, which represents a tridimensional (3D) CAD general view of the entire injection device 1 of the invention as a whole, it is based on a quite straightforward design, using a ball screw linear axis to transform the rotation of the rotor of a high torque servomotor 2 into a linear translation, in order to push on a high-pressure syringe 7. Two separated mobile carts are placed on the axis, with the first one (3) being motorized by the screw while the second one (4) is capable of moving along the axis freely. The two carts 3,4 are linked together through a force sensor 5 in order to measure the linear force running through the assembly 1. A hand maneuvered clamping device 6 has been placed on the free cart 4. It is used to grip a specific syringe piston rod 8 and has been equipped with a low force limit switch to provide an automatic approach during the setup phase. An incremental encoder has also been added between the free cart 4 and the base in order to provide a direct reading of the cart position without being affected by the potential backlash and the flexibility of the kinematic chain.

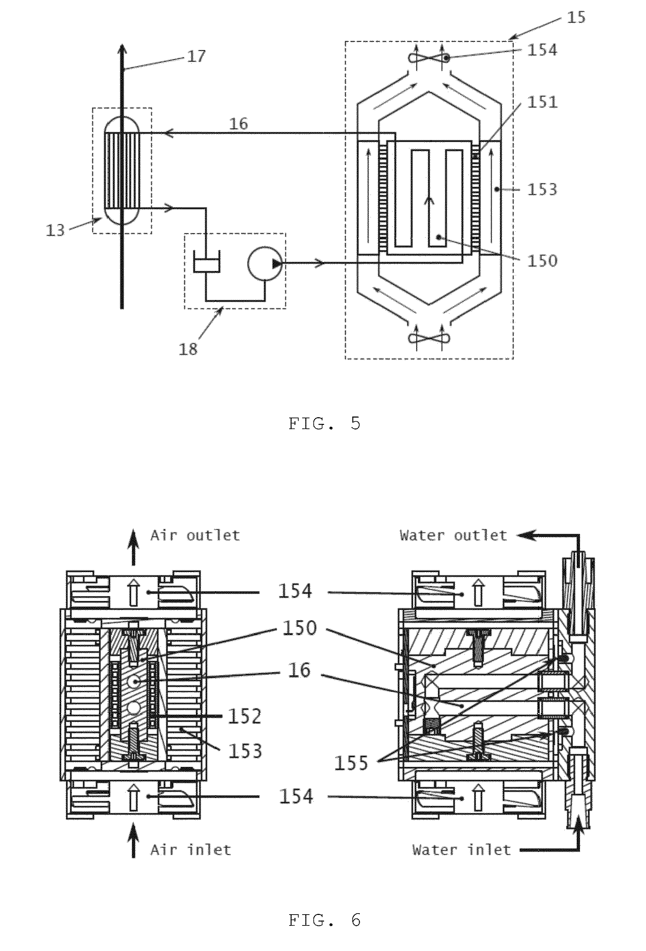

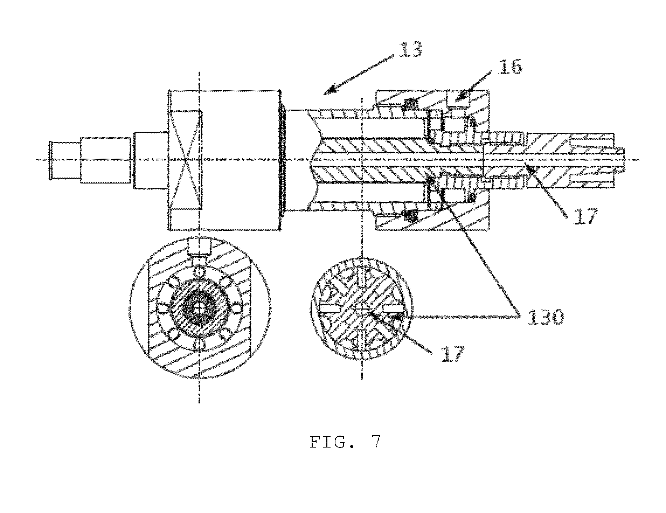

On the fixed part of the device, a mounting base has also been designed to support the syringe. The syringe itself is fixed to the mounting base by using a specific sheath that will be more precisely illustrated and detailed below (see FIGS. 6 and 7 and the corresponding accompanying paragraphs of the text). The syringe 7 is disposable because of the medical nature of its use.

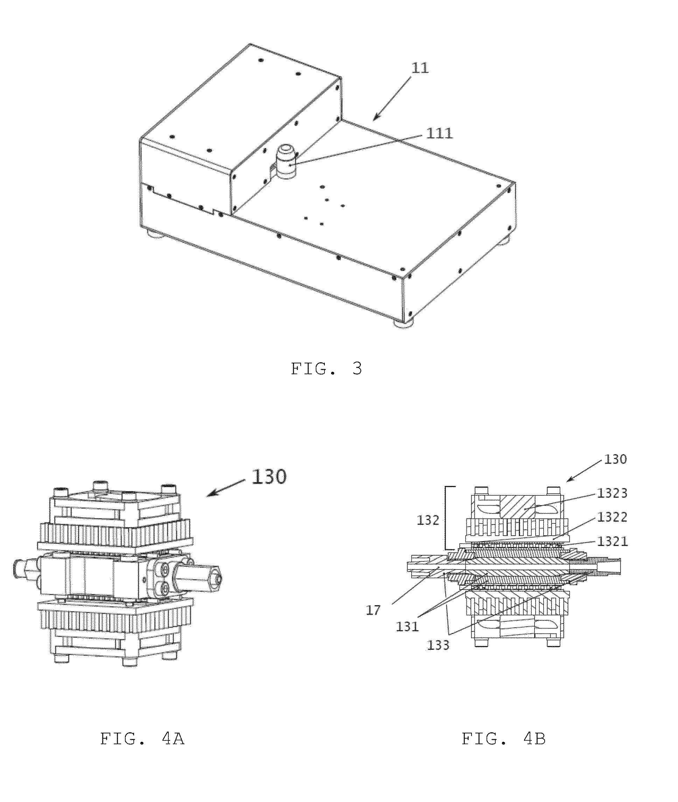

FIG.1 also shows a percutaneous needle 14 connected to the syringe 7 via a pipe 17. An active heat exchanger 13 such as a thermal block (see also figures 4A and 4B) is located on the pipe 17) (surrounding said pipe 17) for dynamic controlled heating and/or cooling of the cement 12 during the injection.

FIG. 2 represents a schematic diagram of the injection device shown on FIG. 1. The motor input 2 provide the displacement of the piston 8 that pushes the cement 12 in the cement channel 17. As shown of the diagram, both the position and force signal are provided by the system.

The overall dimensions of the injection device 1 as a whole are approximately 500 x 100 x 100 mm for a mass of approximately 5.5 kg. It can provide a service load of 2kN. This can generate a pressure of about 100 bar on the cement 12 in the syringe 7, and will allow to inject a fluid with a viscosity up to 2000 Pa.s at a flow rate of 33 $mm^3$/s considering the pressure drop of a 150 x $\varnothing$ 2.5 mm cylinder (equivalent to a typical cement near the end of its solidification and injected in a typical large section injection needle plugged into a short channel).

A master device 11, illustrated on FIG. 3, controls the injection remotely. In order to give to the physician the same feedback as he would have in a manual injection, the master device 11 can provide the force feedback of the pressure applied to the cement 12. Concerning the control of the injection itself, it is a flow rate control, which is more precise and practical than a volumetric control, generally provided by the current known manual systems. It then adds a design constraint to the master device 11 that should return to neutral position when the physician releases the interface because of safety issues. Besides that, the remote control also allows some flexibility as it may have some scaling or non-linear fitting both on the force-feedback and on the control signals, giving the possibility to customize easily both features with the experienced feedback of the practitioner.

The control of the injection is done via a rotating knob 111 located on the master device that returns the pressure information in the form of a force feedback. Should the knob be released by the physician during the injection, an integrated spring returns the interface in a neutral position.

As regards the active thermal regulation, which is realized in the injection device 1 of the invention by an active heat exchanger 13, FIGS. 4A and 4B show a first embodiment of an active heat exchanger 13 consisting in a unique thermal block 130. A thermal insulation (not shown on these figures) wraps the central part of the block 130. The thermal block is composed of

- a central regulated block 131 that is crossed by the cement pipe 17.
- two stacks 132, disposed symmetrically to the regulated block which include :

  - two Peltier modules 1321,
  - two heat sinks 1322,
  - two fans 1323,

- a thermal insulation (not shown on figures 4A and 4B) wrapping the central block 131, which reduces the thermal exchanges between the regulated volume and the ambient air,
- three temperature sensors (not shown on figures 4A and 4B) placed respectively on the central block 131 and on both heat sinks 1322 to measure the temperature of the regulated part and to control both Peltier modules, and
- two Luer lock connections at the extremity of the channel 17 to plug the thermal block 130 to the syringe 7 on one side and to the needle 14 on the other side

The active thermal regulation may also be alternatively realized by a deported active heating/cooling heat exchanger 15 put on a closed fluid circuit 16 consisting for instance in a closed water circuit with an water to cement heat exchanger 13 located on the pipe 17 of the injection device 1, according to a second embodiment of the invention. FIG. 5 represents a schematic diagram of this closed water circuit 16 comprising the deported heat exchanger 15 and the water-to-cement heat exchanger 13 of the injection device 1. The water circulation is powered by a tanking/pumping device 18 placed on the closed loop 16.The advantage of this thermal regulation, in comparison with the thermal block of the first embodiment, is the possibility of doing the same regulation at a remote location, as the thermal block 130 of the first embodiment is both fragile, heavy and space consuming in a critical place such as the surgical area.

The deported heat exchanger 15 of FIGS. 5 and 6 (detailed section of Fig 5) comprises:

- a heat transfer block 150 being crossed by a water circuit 16 connected to the water-to-cement heat exchanger 13,
- Peltier cells 152,
- heat sinks 153, fans 154,temperature sensors 155 placed on the water circuit 16

As the orientation of the fan/sink couple has an impact on the performance on the heat sinks dissipation in free airflow, the fans 154 have been placed in a geometry designed to provide a more efficient forced airflow.

The water circuit 16 shown on FIG.6 also comprises a pumping system 18 that is able to work in reverse direction in order to purge the circuit 16 and thus, to avoid water leakage when the physician unplugs the exchanger 13 from the circuit 16.

The water-to-cement heat exchanger 13 shown on FIG. 7 is built around a finned block 130 whose task is to ensure a proper heat transfer between the cement pipe 17 and the water circuit 16. In this embodiment, it replaces the thermal block 13 presented in FIG. 1 on the cement pipe 17.

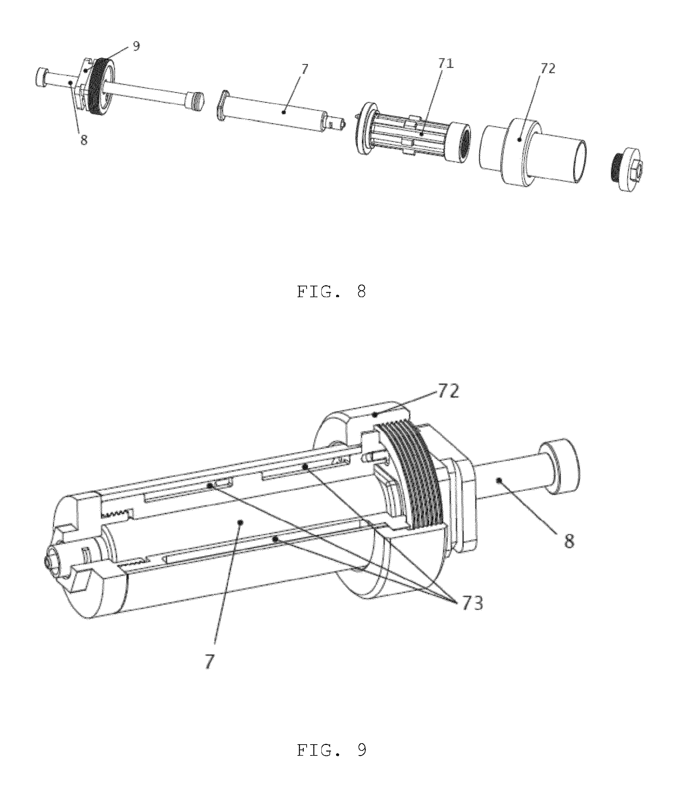

Now turning to the passive thermal exchange, FIGS. 8 and 9 represent a sheath 71 surrounding the syringe 7, according to an embodiment of the invention. In order to design this sheath, several constraints were taken in account:

- it has to resist the service load that may rise up to 2kN
- it should be able to passively exchange thermal energy as to maintain the cement stored within the syringe 7 with the lowest viscosity possible throughout the injection,
- it had to be easily interfaced to the injection device 1 by having a dedicated interface 9 and by allowing a fast and easy locking of the syringe piston 8 to the free cart 4.

As such, the sheath 71 has been machined out of a 316L stainless steel in order to provide a high mechanical resistance and to resist to various chemical products, including biologic fluids and asepsis solutions. The sheath 71 provides some space around the syringe that may filled with an eutectic mixture known for its ability to exchange heat at constant temperatures, thus ensuring that the syringe 71 is kept cool during most of the injection.

The assembly (sheath) is also equipped with a fixation 9 at the back that interfaces with the mounting base 10 on the injector. A nut 72 and screw system is used to put in and extract the disposable syringe that contains the cement.

FIG 8 shows the syringe 71, the syringe sheath 71 (partly disassembled) and the high-pressure piston 8, while FIG. 9 shows a more detailed cutout of the sheath with the syringe 7 in it, where the room 73 for the eutectic gel is visible.

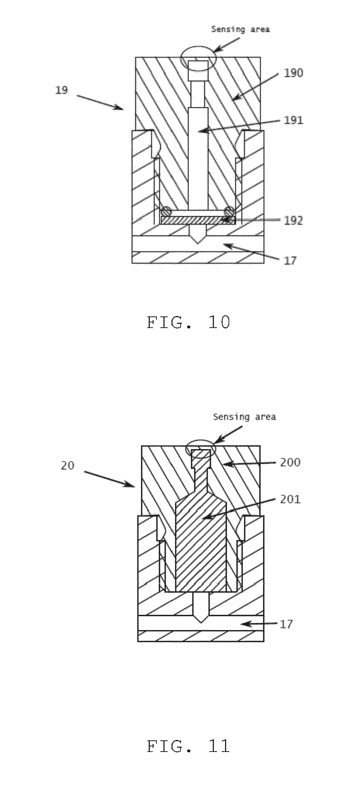

Now turning to pressure measurements of the cement along the cement pipe 17, FIGS. 10 to 12 are cross-sectional schematic views of different embodiments of pressure sensors 19, 20, 21 located on the pipe 17. In order to have a better control on and understanding of the injection procedure, it may be helpful to estimate the intravertebral pressure. This pressure may be used intraoperatively to detect failure during the procedure, such as pressure spikes or drops that may be symptomatic of clogging or leakage.

The best way to measure the intravertebral pressure would be to integrate a pressure sensor at the tip of the needle, but it would be very constraining in terms of design. Thus, in the frame of the present invention, the value of this pressure is obtained indirectly, using a pressure measurement in the cement channel.

Considering the flow of a cement with varying rheological parameters K and n in a cylindrical pipe of length $L_p$ and of radius R, and given the known sensor position distant from the pipe inlet by a distance $L_s$, the Poiseuille flow leads to:

$$Q_S = \left(\frac{\Delta P_s}{L_s}\right)^{1/n_s} \left(\frac{R}{2K_s}\right)^{1/n_s} \left(\frac{\pi n_s R^3}{3n_s + 1}\right)$$

With:

$Q_s$ the volumetric flow at the sensor position

$K_s$ and $n_s$ the rheological parameters of the cement at the sensor position

$\Delta P_s$ the pressure drop between the pipe inlet and the sensor position, obtained by the difference of the pressure value at the sensor ($P_s$) and the pressure value at the inlet ($P_{inlet}$) provided by the injection device:

$$\Delta P_S = P_{inlet} - P_s$$

The same relation also applies between the inlet and the vertebra, giving:

$$Q_v = \left(\frac{\Delta P_v}{L_p}\right)^{1/n_v} \left(\frac{R}{2K_v}\right)^{1/n_v} \left(\frac{\pi n_v R^3}{3n_v + 1}\right)$$

With:

$Q_v$ the volumetric flow at the pipe outlet
$K_v$ and $n_v$ the rheological parameters of the cement entering the vertebra
$\Delta P_v$ the pressure drop between the pipe inlet and the vertebra, hence leading to the intravertebral pressure ($P_v$) through:

$$\Delta P_v = P_{inlet} - P_v$$

Assuming that the flow rate is high enough, the cement viscosity at the sensor is substantially equal to the cement at the outlet of the pipe, leading to $n_s = n_v$, and $K_s = K_v$. Finally, assuming that the flow is incompressive, $Q_s = Q_v$. The two previous equations provide then:

$$\frac{\Delta P_s}{L_s} = \frac{\Delta P_v}{L_p}$$

And hence:

$$P_v = P_{inlet}\left(1 - \frac{L_p}{L_s}\right) + P_s\frac{L_p}{L_s}$$

As shown by the equations, the knowledge of at least one pressure outside the injection pressure is mandatory. But as pressure sensors are too expensive to be integrated as a disposable component, there is a need for a reusable pressure sensor that could be carried over several interventions. Also because of sterility issues, the sensor should not have any internal interface with the cement in order to be cleanable.

For this purpose, reusable pressure sensors have been developed in the frame of the present invention, in which the sensing area of a standard pressure sensor is immersed in an incompressible fluid that would transfer the pressure.

According to a first advantageous embodiment of such a pressure sensor 19 (as shown on FIG.10), it is based on a standard pressure sensor 190 whose channel 191 is filled with water. A flexible interface 192 separates the cement channel 17 and the sensor channel 191. The interface 192 may consist for instance in a cap made out of a flexible silicon compound, such a polydimethylsiloxane (PDMS).

According to a second advantageous embodiment of such a pressure sensor 20 (as shown on FIG.11), it is based on a standard pressure sensor 200 whose channel is filled with an elastomeric compound 201 such as PDMS.

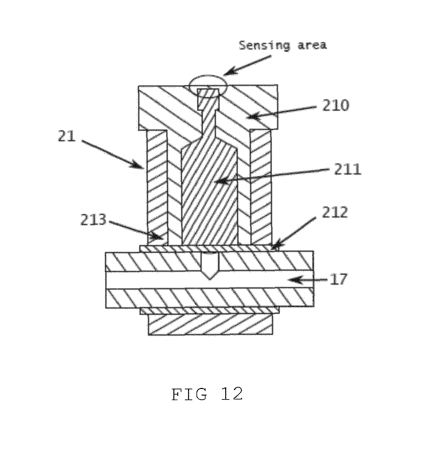

According to a third advantageous embodiment of such a pressure sensor 21 (as shown on FIG.12), it is based on a standard pressure sensor 210 whose channel is filled with an elastomeric compound 211 such as PDMS. The cement pipe 17 is equipped with an elastomeric membrane 212, forming a measure point. The pressure sensor is mounted on a removable bracket 213 that may be plugged on the cement pipe 17, and then removed when the pipe 17 is disposed at the end of the intervention.

## LIST OF THE CITED REFERENCES

[1] A. Gangi, S. Guth, J. Imbert, H. Marin, and J.-L. Dietemann, "Percutaneous vertebroplasty: indications, technique, and results." Radiographics, vol. 23, March 2003.

[2] US 2013/0190680 of Baroud: US patent application filed on March 8, 2013 by the SOCPRA S.E.C and published on July 25, 2013.

[3] US 2009/0062808 of Wolf: US patent application filed on March 2008 by Wolf (as inventor and applicant) and claiming the priority of a provisional application dated September 5, 2007, and published on March 5, 2009.

[4] US 8,523,871 of Truckai et al.: US granted patent filed on April 3, 2008 by Truckai et al. (as inventors and applicants) and claiming the priority of four provisional applications dated April 3, 2007, and granted on October 9, 2008.

## Claims

1. Injection device (1) of curing cement (12) for percutaneous vertebroplasty, said device(1) comprising a system (7,8) for generating volumetric flow of said cement (12), and a pipe (17) connecting said injection device (1) to a percutaneous needle (14),
said injection device (1) being **characterized in that** it further comprises at least one active heat exchanger(s) (13) located on the pipe (17) for dynamic controlled heating and/or cooling of said cement (12) during the injection.

2. Injection device (1) according to claim 1, wherein the system (7,8) for generating volumetric flow comprises:

• a syringe (7) for containing the cement, and
• a piston (8), that is movable inside the syringe (7) for pushing the cement inside the pipe (17) through the syringe outlet.

3. Injection device according to claims 1 or 2, wherein the said at least one active heat exchanger(s) (13) located along the pipe comprises a thermal block (130) with at least one Peltier module (132) mounted on the pipe (17).

4. Injection device (1) according to claim 3, wherein said thermal block (130) comprises:

   - a central regulated block (131) made out of a thermal conducting material with low thermal inertia,
   - at least one stack on the regulated block and including:

     • a Peltier cell (132),
     • one heat sink (133) made out of thermal conducting material with low thermal inertia,
     • a fan (134),

   - a thermal insulation wrapping the central block, and
   - at least one temperature sensor.

5. Injection device according to anyone of claims 1 to 3, further comprising a deported active heat exchanger (15) put on a closed fluid circuit (16) with a fluid-to-cement heat exchanger (13).

6. Injection device according to claim 5, wherein said deported active heat exchanger comprises at least one Peltier module (151).

7. Injection device according to anyone of claims 1 to 4, wherein the heat transfer fluid flowing between the deported active heat exchanger (15) and the fluid-to-cement exchanger (13) is a liquid, a gas or a mixture of liquid and gas, and preferably water.

8. Injection device according to anyone of claims 1 to 7, further comprising a heat exchanger on the syringe (7).

9. Injection device according to claim 8, wherein said heat exchanger on the syringe is a passive heat exchanger, preferably comprising a sheath (71) surrounding the syringe.

10. Injection device according to claim 9, wherein said sheath (71) is filled with a eutectic fluid, preferably a gel.

11. Injection device according to anyone of claims 1 to 10, wherein the said active (13, 15) and/or passive (71) heat exchangers are removable.

12. Injection device according to anyone of claims 1 to 11, further comprising a pressure sensor (19) presenting a sensing area (191), said pressure sensor (19) being located on a defined point of the cement pipe (17).

13. Injection device according to claim 12, wherein the sensing area (191) of said pressure sensor (19) is not in direct contact with the cement (12).

14. Injection device according to claim 12, wherein the pressure of the cement (12) in the pipe (17) is transmitted to the sensing area (191) of said pressure sensor (19) by an intermediary incompressible material, preferably water or an elastomeric material.

15. Injection device according to claim 14, wherein the cement pipe (17) further comprises a sterile elastomeric membrane that is able to transmit the cement pressure to said pressure sensor.

FIG. 1

Force signal

Position signal

FIG. 2

11

111

FIG. 3

130

FIG. 4A

130

132

1323

1322

1321

17

131

133

FIG. 4B

FIG. 5

Air outlet

Water outlet

Air inlet

Water inlet

FIG. 6

FIG. 7

FIG. 8

FIG. 9

Sensing area

19

190

191

192

17

FIG. 10

Sensing area

20

200

201

17

FIG. 11

FIG 12

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2007/148336 A2 (DISC O TECH MEDICAL TECH LTD [IL]; GLOBERMAN OREN [IL]; BEYAR MORDECHA) 27 December 2007 (2007-12-27) <br> * page 5, lines 12-19 * <br> * page 7, lines 7-15 * <br> * page 9, lines 1-21 * <br> * page 13, line 9 - page 15, line 28 * <br> * figures 1B, 2-5 * | 1-7,11 | INV. <br> A61B17/88 <br> A61F2/46 |
| X | US 2008/269761 A1 (TRUCKAI CSABA [US] ET AL) 30 October 2008 (2008-10-30) <br> * page 4, paragraph 58 - page 8, paragraph 86 * <br> * figures 1-4, 5C, 5D, 8A, 8B, 10A, 10B, 11 * | 1-3,5,7, 11 | |
| X <br> Y | US 2012/239049 A1 (TRUCKAI CSABA [US] ET AL) 20 September 2012 (2012-09-20) <br> * page 13, paragraph 136 - page 14, paragraph 144 * <br> * figures 21, 22 * | 1-4,8,9, 12 <br> 13-15 | |
| Y <br> A | US 2009/093818 A1 (BAROUD GAMAL [CA]) 9 April 2009 (2009-04-09) <br> * page 3, paragraph 47-48 * <br> * page 4, paragraph 64 * <br> * figures 1, 5B * | 13-15 <br> 1,2,12 | TECHNICAL FIELDS SEARCHED (IPC) <br><br> A61B <br> A61F |

The present search report has been drawn up for all claims

| Place of search | Date of completion of the search | Examiner |
|---|---|---|
| Berlin | 10 December 2015 | Kakoullis, Marios |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 15 30 5974

This annex lists the patent family members relating to the patent documents cited in the above-mentioned European search report.
The members are as contained in the European Patent Office EDP file on
The European Patent Office is in no way liable for these particulars which are merely given for the purpose of information.

10-12-2015

| Patent document cited in search report | | Publication date | Patent family member(s) | | Publication date |
|---|---|---|---|---|---|
| WO 2007148336 | A2 | 27-12-2007 | ES 2543346 T3 | | 18-08-2015 |
| | | | US 2014148866 A1 | | 29-05-2014 |
| | | | WO 2007148336 A2 | | 27-12-2007 |
| US 2008269761 | A1 | 30-10-2008 | US 2008269761 A1 | | 30-10-2008 |
| | | | US 2013226142 A1 | | 29-08-2013 |
| | | | US 2014303634 A1 | | 09-10-2014 |
| | | | WO 2008137428 A2 | | 13-11-2008 |
| US 2012239049 | A1 | 20-09-2012 | US 2007118144 A1 | | 24-05-2007 |
| | | | US 2007162043 A1 | | 12-07-2007 |
| | | | US 2007191858 A1 | | 16-08-2007 |
| | | | US 2007233148 A1 | | 04-10-2007 |
| | | | US 2009012525 A1 | | 08-01-2009 |
| | | | US 2012239049 A1 | | 20-09-2012 |
| | | | WO 2007028120 A2 | | 08-03-2007 |
| US 2009093818 | A1 | 09-04-2009 | CA 2648283 A1 | | 18-10-2007 |
| | | | EP 2010266 A1 | | 07-01-2009 |
| | | | US 2009093818 A1 | | 09-04-2009 |
| | | | US 2013190680 A1 | | 25-07-2013 |
| | | | WO 2007115402 A1 | | 18-10-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8523871 B
- US 20130190680 A
- US 20090062808 A

**Non-patent literature cited in the description**

- **A. GANGI ; S. GUTH ; J. IMBERT ; H. MARIN ; J.-L. DIETEMANN.** Percutaneous vertebroplasty: indications, technique, and results. *Radiographics,* 23 March 2003